# EUROPÄISCHE PATENTSCHRIFT

(11) **EP 2 895 453 B1**
(45) Veröffentlichungstag und Bekanntmachung des Hinweises auf die Patenterteilung: **07.11.2018**
(21) Anmeldenummer: 13762988.7
(22) Anmeldetag: 10.09.2013
(51) Int. Cl.: C07C 41/09, B01J 19/00, C07C 43/04

(54) **VERFAHREN ZUR HERSTELLUNG VON DIMETHYLETHER UND DAFÜR GEEIGNETE VORRICHTUNG**
METHOD FOR PRODUCING DIMETHYL ETHER AND DEVICE SUITABLE THEREFOR
PROCÉDÉ DE PRÉPARATION D'ÉTHER DIMÉTHYLIQUE ET DISPOSITIF APPROPRIÉ AU PROCÉDÉ

(30) Priorität: 15.09.2012 DE 102012018341
(43) Veröffentlichungstag der Anmeldung: 22.07.2015
(73) Patentinhaber: thyssenkrupp Industrial Solutions AG, 45143 Essen (DE)
(72) Erfinder: BAUER, Melanie, 45147 Essen (DE); KÖMPEL, Harald, 61118 Bad Vilbel (DE); SCHULZ, Alexander, 60439 Frankfurt (DE)
(74) Vertreter: thyssenkrupp Intellectual Property GmbH
(86) Internationale Anmeldenummer: PCT/EP2013/002702
(87) Internationale Veröffentlichungsnummer: WO 2014/040719

(56) Entgegenhaltungen:
- WO-A1-2006/041253
- WO-A2-2013/041516
- WO-A2-2013/041516
- CN-A- 101 903 323
- DE-A1-102011 114 228

## Beschreibung

Die Erfindung betrifft ein Verfahren zur Herstellung von Dimethylether durch katalytische Dehydratisierung von Methanol und destillative Aufarbeitung des Dehydratisierungsproduktes, wobei die katalytische Dehydratisierung in mindestens zwei in Reihe geschalteten Reaktionsstufen erfolgt, von denen zumindest die erste Reaktionsstufe adiabat betrieben wird und wobei zumindest zwischen der ersten und der zweiten Reaktionsstufe eine Kühlung des Reaktionsproduktes erfolgt.

Dimethylether (nachstehend auch "DME" genannt) wird auf vielen Gebieten in der industriellen Produktion sowie vom privaten Verbraucher eingesetzt. Bespiele dafür sind der Einsatz als Treibmittel z.B. für Haarspray, oder als Einsatzstoff für chemische Synthesen z.B. zur Herstellung von Dimethylsulfat oder von leichten Olefinen (Ethylen, Propylen, Butenen). Darüber hinaus ist DME ein emissionsarmer Brennstoff, der alternativ zu Flüssiggas aus Rohöl ("LPG") eingesetzt wird und dieses langfristig ersetzen kann. Auch der Einsatz als emissionsarmer Treibstoff für Dieselfahrzeuge wurde in mehreren Ländern erfolgreich erprobt. DME wird üblicherweise aus Synthesegas (H₂ und CO) hergestellt, das durch Reformieren von Erdgas oder durch Vergasung von Kohle oder Feststoffen gewonnen wird.

Die Herstellung von Dimethylether erfolgt dann entweder durch Direktsynthese aus Synthesegas oder zweistufig über die Methanolsynthese und die anschließende Umsetzung des Methanols zu DME und Wasser. Die heute weltweit produzierte Menge an DME wird fast ausschließlich aus Methanol hergestellt. Die zweite Stufe dieser "indirekten" DME-Synthese, die Herstellung von DME aus Methanol, beruht auf der bekannten reaktionstechnischen Auslegungsbasis der Umwandlung von Methanol zu DME und Wasser in der Gasphase an einem sauren Katalysator, beispielsweise an Al₂O₃, in einem einstufigen Festbett Reaktor. Dabei findet folgende chemische Reaktion statt:

2 CH₃OH ↔ CH₃OCH₃ + H₂O,

ΔH = - 24 kJ/mol

Die Wärme der exothermen Reaktion wird dabei entweder durch Kühlung im Reaktor abgeführt oder das dampfförmige Einsatzmethanol wird bei adiabater Reaktionsführung durch die Wärme des Reaktionsproduktes in einem Einsatz-Produkt-Wärmetauscher überhitzt. Im Falle eines gekühlten Reaktors wird dieser typischerweise als Rohrreaktor ausgeführt, wobei die chemische Reaktion in den mit Katalysator befüllten Rohren stattfindet und gleichzeitig die Reaktion durch das dampfförmige Einsatzmethanol gekühlt wird, das auf der Mantelseite des Reaktors geführt wird und dort durch die Reaktionswärme weiter vorgewärmt wird.

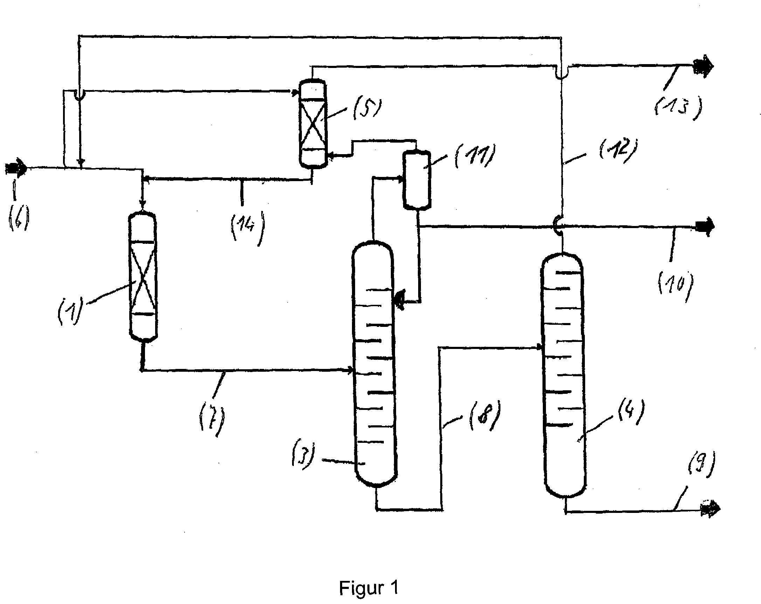

Die im folgenden als "Stand-der-Technik-DME-Verfahren" beschriebene Version des methanolbasierten DME-Verfahrens beruht auf der Verwendung eines DME-Reaktors. An den DME-Reaktor schließt üblicherweise eine Produktaufarbeitung mit zwei Rektifikationskolonnen, einer DME-Kolonne und einer Methanol-Kolonne zur Abtrennung von nicht umgesetztem Einsatzmethanol von Wasser, sowie einem AbgasWäscher an. Dieses DME-Verfahren wird in Figur 1 dargestellt.

Das Stand-der-Technik-DME-Verfahren beinhaltet üblicherweise eine aufwändige Wärmeintegration, wobei das heiße Reaktionsprodukt zum Aufwärmen des Einsatz-Methanols sowie zum Betrieb von Aufkochern oder zum Aufheizen von umgepumpten Strömen in der Nähe des Sumpfes einer der Kolonnen genutzt wird.

Bei großtechnischen Verfahren wird ständig nach einer Verbesserung der Verfahrens-ökonomie gesucht. Mögliche Verbesserungen können die Energieeffizienz, geringere Reinheitsanforderungen an Einsatzstoffe, höhere Produktreinheit, die Produktivität und/oder die verwendeten Apparaturen betreffen.

Die ältere, nicht vorveröffentlichte DE 10 2011 114 228 A1 offenbart einen gekühlten Reaktor zur Herstellung von Dimethylether aus Methanol durch heterogen katalysierte Dehydratisierung. Es wird ein Reaktor eingesetzt, in dem zunächst eine adiabate Aufheizung durch die in der Startzone freigesetzte Reaktionswärme erfolgt, wodurch die Reaktionsgeschwindingkeit auf technisch akzeptable Werte erhöht wird. Eines der dargestellten Reaktordesigns umfasst mehrere in Reihe geschaltete Katalysatorbetten. Die Aufarbeitung des Reaktionsproduktes Dimethylether wird nicht offenbart.

Aus US 2009/0023958 A1 ist ein Verfahren zur Herstellung von Dimethylether aus Methanol in einem adiabatisch betriebenen Reaktor bekannt, in dem zwei in Reihe geschaltete Katalysatorbetten angeordnet sind. Das Verfahren ist durch den Einsatz ausgewählter Katalysatoren in den Katalysatorbetten charakterisiert.

US 4,560,87 A offenbart ein weiteres Verfahren zur Herstellung von Dimethylether sowie die Aufarbeitung des dabei entstandenen Produktes. Der erhaltene Dimethylether entsteht in guter Ausbeute und fällt in hoher Reinheit an.

Aus der Chinesischen Schrift CN 101903323 A ist ein Verfahren zur Herstellung von Dimethylether durch katalytische Dehydratisierung von Methanol und destillative Aufarbeitung des Dehydratisierungsprodukts bekannt, wobei die Dehydratisierung in mindestens zwei in Reihe geschalteten Reaktionsstufen erfolgt, von denen zumindest die erste Reaktionsstufe adiabat betrieben wird und wobei zumindest zwischen der ersten und der zweiten Reaktionsstufe eine Kühlung des Reaktionsprodukts erfolgt. Bei diesem bekannten Verfahren wird der Dimethylether und somit das Produkt als Kühlflüssigkeit eingesetzt. Alternativ kann Wasser als Kühlflüssigkeit verwendet werden. Da somit als Kühlmedium im Prinzip das Produkt der Reaktion eingesetzt wird, wobei es sich bei dieser Umsetzung um eine Gleichgewichtsreaktion handelt, wird durch diese Maßnahme der Gleichgewichtsumsatz reduziert.

Aufgabe der vorliegenden Erfindung ist die Bereitstellung eines verbesserten Verfahrens und einer dafür geeigneten Anlage zur Herstellung von Dimethylether, die sich durch eine hohe Produktivität auszeichnen.

Die DME-Synthese ist eine Gleichgewichtsreaktion. Sie ist nicht oder nur unwesentlich vom Druck abhängig. Das Gleichgewicht kann durch eine niedrige Arbeitstemperatur in Richtung der DME-Bildung verlagert werden. Gleichzeitig erfordert allerdings die Kinetik der katalytischen Reaktion eine Mindestarbeitstemperatur, damit die chemische Reaktion zündet und stabil verläuft.

Um einen hohen Umsatz der Gleichgewichtsreaktion zu erreichen ist es also vorteilhaft, mit einer möglichst niedrigen Reaktortemperatur zu arbeiten, welches eine relativ geringe Reaktoraustrittstemperatur zur Folge hat.

Kleine DME-Anlagen z.B. zur Herstellung von reinem DME als Treibmittel haben meistens einen gekühlten Reaktor. Solche Anlagen haben üblicherweise Kapazitäten von 10.000 bis 40.000 t pro Jahr. Der gekühlte Reaktor, ausgeführt als Rohrreaktor mit Kühlung durch Methanoldampf auf der Mantelseite des Apparates, ist bei kleinen bis mittleren Anlagenkapazitäten wirtschaftlich sinnvoll.

Größere DME-Anlagen zur Herstellung von Brennstoff-DME als LPG- oder Diesel-Ersatz haben üblicherweise Kapazitäten von größer als 100.000 t pro Jahr. Das Design solch großer Anlagen ist seit etwa zehn Jahren bekannt, während kleine Anlagen zur Erzeugung von Rein-DME bereits seit über dreißig Jahren gebaut werden. In der Baugröße der Brennstoff-DME-Anlagen sind Rohrreaktoren sehr teuer, wegen der großen Zahl an Rohren und weil bei den größten Kapazitäten zwei Rohrreaktoren parallel vorgesehen werden müssen. Industriell wird daher bei solch großen Anlagenkapazitäten aus Gründen niedriger Investitionskosten ein adiabater Festbettreaktor verwendet, der beispielsweise die Form eines Schachtreaktors aufweisen kann. Nachteilig ist bei der adiabaten Fahrweise, dass sich innerhalb des Reaktors die Temperatur der Reaktionsmischung typischerweise um mehr als 100 °C erhöht. Dadurch verschiebt sich das Reaktionsgleichgewicht im Vergleich mit einem gekühlten Reaktor, der eine niedrigere Austrittstemperatur aufweist, zu einem niedrigeren Methanolumsatz. Als Folge muss in der Methanol-Kolonne mehr nicht umgesetztes Methanol zurück gewonnen werden, was die Investitionskosten und die Betriebsmittelkosten dieser Kolonne deutlich erhöht.

Um hier Abhilfe zu schaffen, wurde erfindungsgemäß das übliche Konzept eines adiabaten Reaktors verbessert durch eine Reaktionsführung von mindestens zwei in Reihe geschalteten Reaktionsstufen, von denen zumindest die erste adiabat betrieben wird und wobei das Reaktionsprodukt zwischen den beiden Reaktionsstufen gekühlt wird.

Die vorliegende Erfindung betrifft ein Verfahren zur Herstellung von Dimethylether durch katalytische Dehydratisierung von Methanol und destillative Aufarbeitung des Dehydratisierungsproduktes, bei dem die katalytische Dehydratisierung in mindestens zwei in Reihe geschalteten Reaktionsstufen erfolgt, von denen zumindest die erste Reaktionsstufe adiabat betrieben wird und wobei zumindest zwischen der ersten und der zweiten Reaktionsstufe eine Kühlung des Reaktions-produktes erfolgt, wobei drei in Reihe geschaltete adiabatisch betriebene Reaktionsstufen vorgesehen sind, zwischen der ersten und zweiten Reaktionsstufe flüssiges Methanol als Kühlmittel dem Reaktionsgemisch zugesetzt wird und zwischen der zweiten und dritten Reaktionsstufe die Kühlung des Reaktionsgemisches durch einen Wärmetauscher erfolgt, der vorzugsweise Dämpfe des für die Dehydratisierung eingesetzten Methanols als Kühlmedium führt.

Es kann aber auch eine höhere Anzahl von in Reihe geschalteten Reaktionsstufen vorgesehen sein, von denenbevorzugt sämtliche Reaktionsstufen adiabatisch betrieben werden.

In einer bevorzugten Verfahrensvariante besteht zumindesten eine Reaktionsstufe aus mehreren parallel geschalteten Reaktoren. Besonders bevorzugt werden in sämtlichen in Reihe geschalteten Reaktionsstufen jeweils mehrere parallel geschaltete Reaktoren eingesetzt.

Zumindest zwischen der ersten und der zweiten Reaktionsstufe erfolgt eine Kühlung des Reaktionsgemisches. Vorzugsweise findet zwischen jeder Reaktionsstufe eine Kühlung des Reaktionsgemisches statt.

Die Temperatur in den Reaktionsstufen des erfindungsgemäßen Verfahrens ist im Vergleich zur Temperatur in den adiabatisch betriebenen Reaktoren des Standes der Technik herabgesetzt. Typischerweise bewegt sich die Temperatur in den erfindungsgemäß in Reihe geschalteten Reaktionsstufen jeweils im Bereich von 200 bis 400°C, vorzugsweise von 250 bis 370°C.
Zwischen einzelnen Reaktionsstufen wird das Reaktionsgemisch gekühlt. Innerhalb einer adiabatischen Reaktionsstufe steigt die Reaktionstemperatur an, da das Verfahren exotherm ist. In der Regel kühlt man das Reaktionsgemisch nach Durchlauf einer adiabatischen Reaktionsstufe so weit ab, dass dessen Temperatur etwa der Eintrittstemperatur des Reaktionsgemisches in der vorangegangenen adiabatischen Reaktionsstufe entspricht. Im Falle einer nicht adiabatisch betriebenen Reaktionsstufe, beispielsweise einer isotherm betriebenen Reaktionsstufe wird das Reaktionsgemisch unter die Eintrittstemperatur abgekühlt und somit die DME-Bildung begünstigt. Bevorzugt wird die Temperatur des Reaktionsgemisches zwischen den Reaktionsstufen auf 200 bis 300°C abgekühlt.

Das Abkühlen des Reaktionsgemisches kann durch den Einsatz von Wärmetauschern und/oder durch Zugabe von Kühlflüssigkeit direkt in das Reaktionsgemisch ("Quenchen") erfolgen.

Als Kühlflüssigkeit eignen sich Methanol, DME und/oder Wasser, wobei in den ersten Reaktionsstufen vorzugsweise flüssiges Methanol eingesetzt wird, und in der letzten

Reaktionsstufe vorzugsweise flüssiges DME oder eine DME enthaltende Flüssigkeit. Die Kühlflüssigkeit wird in das gasförmige Reaktionsgemisch zwischen den Reaktionsstufen eingebracht, z.B. eingedüst, und bewirkt durch Verdampfen eine effektive Kühlung des Reaktionsgemisches.

Als Wärmetauscher eignen sich alle bekannten Typen, wie Spiralwärmetauscher, Rohrbündelwärmetauscher und Plattenwärmetauscher. Diese werden vorzugsweise mit Flüssigkeiten beschickt, welche aus der DME-Anlage stammen und eine Kühlung des heißen Reaktionsgemisches bewirken können. So lässt sich beispielsweise Methanol aus der Methanol-Kolonne als Kühlmittel für den oder die Wärmetauscher einsetzen.

Die verschiedenen Reaktionsstufen des erfindungsgemäßen Verfahrens können durch drei in Reihe geschaltete Reaktoren realisiert werden, die adiabatisch betrieben wird. Dabei erfolgt zumindest zwischen den ersten beiden Reaktoren eine Kühlung des Reaktionsgemisches.

In einer alternativen Variante des erfindungsgemäßen Verfahrens können die verschiedenen Reaktionsstufen in einem Reaktor realisiert werden, wobei die Reaktionsstufen adiabatisch betrieben werden. Vorzugsweise wird ein adiabatisch betriebener Reaktor eingesetzt, der drei in Reihe geschaltete Katalysator-schüttungen aufweist. Zumindest zwischen der ersten und der zweiten Katalysator-schüttung, vorzugsweise zwischen sämtlichen Katalysatorschüttungen, ist jeweils ein Wärmetauscher zur Zwischenkühlung des Reaktionsgemisches aus dem stromaufwärts liegenden Katalysatorbett geschaltet und/oder zwischen zumindest den ersten beiden Katalysatorschüttungen, vorzugsweise zwischen sämtlichen Katalysatorschüttungen wird Kühlflüssigkeit in das Reaktionsgemisch eingedüst.

Bevorzugt eingesetzte Reaktoren sind adiabatisch betriebene Festbettreaktoren.

Bei nicht adiabatischem Betrieb werden vorzugsweise Rohrbündelreaktoren oder Wirbelschichtreaktoren eingesetzt.

Als Wärmetauscher können sämtliche bekannten Typen eingesetzt werden. Beispiele dafür sind Rohrbündelwärmetauscher, Spiralwärmetauscher oder Plattenwärmetauscher.

In dem erfindungsgemäßen Verfahren erfolgt die Kühlung des Reaktionsproduktes zwischen den Reaktionsstufen durch einen Wärmetauscher, der Dämpfe des für die Dehydratisierung eingesetzten Methanols als Kühlmedium führt.

In einer besonders bevorzugten Variante des erfindungsgemäßen Verfahrens sind drei in Reihe geschaltete adiabatisch betriebene Reaktionsstufen vorgesehen, zwischen der ersten und zweiten Reaktionsstufe wird flüssiges Methanol als Kühlmittel dem Reaktionsgemisch zugesetzt und zwischen der zweiten und dritten Reaktionsstufe erfolgt die Kühlung des Reaktionsgemisches durch einen Wärmetauscher, der vorzugsweise Dämpfe des für die Dehydratisierung eingesetzten Methanols als Kühlmedium führt.

Diese Ausführungsform des erfindungsgemäßen Verfahrens wird vorzugsweise in einem adiabatisch betriebenen Reaktor durchgeführt, der drei hintereinander geschaltete Katalysatorbetten aufweist, zwischen dem ersten und zweiten Katalysatorbett eine Vorrichtung zum Einbringen von flüssigem Methanol in das Reaktionsgemisch und zwischen dem zweiten und dritten Katalysatorbett einen Wärmetauscher, vorzugsweise einen Plattenwärmetauscher zum Kühlen des Reaktionsgemisches vor Eintritt in das dritte Katalysatorbett.

Im erfindungsgemäßen Verfahren können die an sich üblichen für die Dehydratisierung von Methanol verwendeten Katalystoren eingesetzt werden. Bevorzugt wird für die Dehydratisierung ein saurer und fester Katalysator verwendet, vorzugsweise Aluminiumoxid. Alternativ zu Aluminiumoxid können auch andere feste saure Katalysatoren eingesetzt werden, beispielsweise Aluminosilikate, wie Zeolithe oder Titandioxid oder Aluminotitanate.

Durch die oben beschriebene Führung der Reaktion wird das Reaktionsgleichgewicht bei einer niedrigen Reaktoraustrittstemperatur, vergleichbar mit der eines gekühlten Reaktors, eingestellt und somit wird ein höherer Methanolumsatz und eine geringere Rückführmenge an Methanol erzielt. Die Leistung der Methanolkolonne wird dadurch deutlich verringert.

In einer bevorzugten Ausführungsform des erfindungsgemäßen Verfahren wird das aus der letzten Reaktionsstufe stammende Reaktionsgemisch in einer DME-Kolonne aufgearbeitet, indem Dimethylether aus dem Reaktiongemisch destillativ abgetrennt wird und ein Sumpfprodukt anfällt, wobei das Sumpfprodukt aus der DME-Kolonne in eine Methanol-Kolonne überführt wird, und dort destillativ in einen Methanolstrom und ein Wasser enthaltendes Sumpfprodukt aufgetrennt wird. Der erhaltene Methanolstrom wird vorzugsweise in eine oder mehrere der DME-Reaktionsstufen zurückgeführt.

Das erfindungsgemäße Verfahren besitzt im Vergleich mit dem Stand der-Technik-DME-Verfahren eine Reihe von Vorteilen. Im erfindungsgemäßen Verfahren wird in der Methanol-Kolonne des zweistufigen Reaktionskonzepts bis zu etwa 30 % weniger Methanol abdestilliert. Die Rückführmenge an Methanol reduziert sich also aufgrund des höheren Methanolumsatzes der zweistufigen Reaktionsführung auf bis zu etwa 70 % des im Stand-der-Technik-DME-Verfahren anfallenden Wertes. Dadurch kann beim erfindungsgemäßen Verfahren der Durchmesser der Methanol-Kolonne verkleinert werden und es können Kosten an Betriebsmitteln in der Stofftrennung eingespart werden.

Das erfindungsgemäße Verfahren resultiert in einer im Vergleich mit anderen adiabatischen Verfahren niedrigeren Reaktoraustrittstemperatur und lässt daher einen höheren Methanol-Gleichgewichtsumsatz zu, beispielsweise von bis zu 88 %.

Das erfindungsgemäße Verfahren führt also zusammenfassend zu:
- einer geringeren Methanol-Kreislaufmenge (etwa 30 % kleiner)
- und somit zu kleineren Ausrüstungen (beispielsweise wird der Durchmesser der Methanol Kolonne um etwa 17 % kleiner)
- und zu niedrigeren Betriebskosten für die Stofftrennung (Einsparung an Kühlwasser von etwa 20 %, Einsparung an Dampf von etwa 40 %).

Mehrkosten treten infolge des Einsatzes eines zweiten Reaktors und infolge von etwas Mehrbedarf an Katalysator auf.

Bei Betrachtung der Gesamtwirtschaftlichkeit einer großen DME-Anlage von 800.000 t pro Jahr, auf Basis des Vergleichs von Investitionskosten und Betriebskosten der Anlage über eine Lebensdauer von 20 Jahren, ist die zweistufige Reaktionsführung wirtschaftlicher als die "standardmäßige" DME-Anlage mit einem adiabaten Reaktor.

Produkterlös und Rohstoffkosten sind bei beiden Varianten gleich, da die DME- und Methanol-Mengenströme identisch sind. Die höhere Wirtschaftlichkeit ergibt sich also durch die niedrigeren Gesamtkosten.

Die Erfindung betrifft auch die Verwendung einer Vorrichtung in dem Verfahren zur Herstellung von Dimethylether durch katalytische Dehydratisierung von Methanol umfassend die Elemente:
A) mindestens zwei in Reihe geschaltete DME-Reaktoren (1a, 1b), von denen mindestens der erste DME-Reaktor adiabatisch betrieben wird,
B) zwischen mindestens dem ersten und dem zweiten DME-Reaktor angeordnete Kühlvorrichtung (2) für das Reaktionsgemisch aus dem stromaufwärts zur Kühlvorrichtung (2) gelegenen Reaktor (1a),
C) mit dem letzten Reaktor (1b) verbundene DME-Kolonne (3) zur Abtrennung des Dimethylethers aus dem Reaktionsgemisch, und
D) mit dem Sumpf der DME-Kolonne (3) verbundene Methanol-Kolonne (4) zur Auftrennung des vom Dimethylether befreiten Reaktionsgemischs in Methanol und Wasser.

Eine alternative Ausführungsform der Erfindung betrifft die Verwendung einer Vorrichtung in dem Verfahren zur Herstellung von Dimethylether durch katalytische Dehydratisierung von Methanol umfassend die Elemente:
A') mindestens einen DME-Reaktor, in dem mindestens zwei in Reihe geschaltete Reaktionsstufen angeordnet sind, von denen zumindest die erste Reaktionsstufe adiabatisch betrieben wird,
B') zwischen mindestens der ersten und der zweiten Reaktionsstufe angeordnete Kühlvorrichtung (2) für das Reaktionsgemisch aus der stromaufwärts zur Kühlvorrichtung gelegenen Reaktionsstufe,
C') mit der letzten Reaktionsstufe verbundene DME-Kolonne (3) zur Abtrennung des Dimethylethers aus dem Reaktionsgemisch, und
D) mit dem Sumpf der DME-Kolonne (3) verbundene Methanol-Kolonne (4) zur Auftrennung des vom Dimethylether befreiten Reaktionsgemischs in Methanol und Wasser.

In einer bevorzugten Ausführungsform weist die erfindungsgemäße Vorrichtung einen adiabat betriebenen DME-Reaktor auf, in dem zwei in Reihe geschaltete Katalysatorschüttungen vorgesehen sind, sowie eine Kühlvorrichtung , die zur Zwischenkühlung des Reaktionsgemisches aus dem stromaufwärts liegenden Katalysatorbett dient. Bei dem adiabat betriebenen Reaktor handelt es sich insbesondere um einen vertikalen Schachtreaktor. Bei der Kühlvorrichtung (2) handelt es sich um Wärmetauscher und/oder um Vorrichtungen zum Einleiten von Kühlflüssigkeit in das Reaktionsgemisch.

In einer weiteren bevorzugten Ausführungsform wird in der erfindungsgemäßen Vorrichtung Methanol aus der Methanol-Kolonne (4) in den ersten adiabatisch betriebenen DME-Reaktor (1a) oder in die erste adiabatisch betriebene Reaktionsstufe des DME-Reaktors zurückgeführt.

In einer weiteren bevorzugten Ausführungsform wird in der erfindungsgemäßen Vorrichtung als Kühlvorrichtung (2) ein Wärmetauscher eingesetzt, der Dämpfe des für die Dehydratisierung eingesetzten Methanols als Kühlmedium führt.

Die Investitionskosten des Falls mit zweistufiger Reaktionsführung können weiter gesenkt werden, indem die zwei Reaktionsstufen nicht durch zwei Apparate (zwei Reaktoren), sondern durch einen Reaktor realisiert werden. Ein solch integrierter vertikaler Schachtreaktor beinhaltet zwei oder noch mehr Katalysatorschüttungen und ein oder mehrere Zwischenkühlungen mit Hilfe eines oder mehrerer eingebauter Wärmetauscher, vorzugsweise von Plattenwärmetauschern, oder durch Einleiten von Kühlflüssigkeit, vorzugsweise von Methanol. Besonders bevorzugt werden die Wärmetauscher mit Dämpfen des Einsatzmethanols als Kühlmedium beschickt.

Die Figuren 1 bis 2 beschreiben beispielhaft und schematisch ein Verfahren des Standes der Technik und eine Variante des erfindungsgemäßen Verfahrens.

In Figur 1 ist das bekannte DME-Verfahren schematisch dargestellt. Das Einsatz-Methanol (6) wird verdampft und überhitzt und dann dem DME-Reaktor (1) mit einer Temperatur von mindestens 250 °C zugeführt. Die Gleichgewichtsreaktion von Methanol zu DME und Wasser findet an einem sauren Katalysator statt, bei einem Druck von ca. 12 - 14 bar(a) und mit einem Methanolumsatz von ca. 83 %. Das Reaktionsprodukt (7) verlässt den DME-Reaktor (1) mit ca. 370 °C und wird durch Wärmeintegration abgekühlt, beispielsweise zunächst durch Wärmeaustausch mit dem Einsatzmethanoldampf und dann durch Beheizung eines Aufkochers oder durch Aufheizen von umgepumpten Strömen in der Nähe des Sumpfes einer der Kolonnen. Das so abgekühlte Reaktionsprodukt (7) wird in die DME-Kolonne (3) geleitet. In dieser DME-Kolonne (7) wird das DME bei einem Druck von ca. 10 - 12 bar(a) als flüssiges Kopfprodukt abgetrennt, bei Verwendung von Kühlwasser im Kopfkondensator, und einem Rückflußbehälter (11) zugeführt. Diesem wird flüssiges DME-Produkt (10) entnommen und aus der Anlage ausgeschleust bzw. ein Teil davon wird in die DME-Kolonne zurückgeführt. Die Gasphase aus dem Rückflußbehälter (11) wird einem DME-Absorber (5) zugeführt. Außerdem wird dem DME-Absorber (5) ein Teil des Einsatz-Methanols (6) zugeführt. Im DME-Absorber (5) werden nichtkondensierbare Gase (13), bestehend aus einer geringen Menge an Spaltgas (H₂, CO, CO₂ und CH₄) und DME, mit Methanol gewaschen, um das DME zurückzugewinnen und das flüssige Produkt (14) aus dem DME-Absorber (5) wird dem DME-Reaktor (1) zugeführt. Das Sumpfprodukt (8) der DME Kolonne (3) wird in der Methanol-Kolonne (4), die bei geringem Überdruck betrieben wird, in Methanol (12) und Wasser (9) aufgetrennt. In der Methanol-Kolonne (4) wird das nicht umgesetzte Methanol (12) zurückgewonnen, um dieses erneut dem Prozess zu zuführen. Die verbliebene Wassermenge im zurückgeführten Methanol ist Gegenstand einer Optimierung, da sie Auswirkung auf die Kosten von Methanol-Kolonne (4) und DME-Reaktor (1) hat. Ein höherer Wassergehalt im Kreislaufmethanol beeinflusst über das Reaktionsgleichgewicht den Umsatz des Methanols in einer ungünstigen Weise und bedingt zudem, dass die chemische Reaktion mit einer höheren Eintrittstemperatur gefahren werden muss, was wiederum ungünstige Auswirkungen auf das Gleichgewicht hat, da bei höherer Temperatur der Methanolumsatz geringer wird.

Das geschilderte Verfahren wird in Ullmann's Encyclopedia of Industrial Chemistry, Fifth Completely Revised Edition, Volume A6, Seite 541 bis 544, aus dem Jahre 1987 beschrieben und auch in zahlreichen Patentschriften wie z.B. US 4,802,958 und EP 0 270 852 A2.

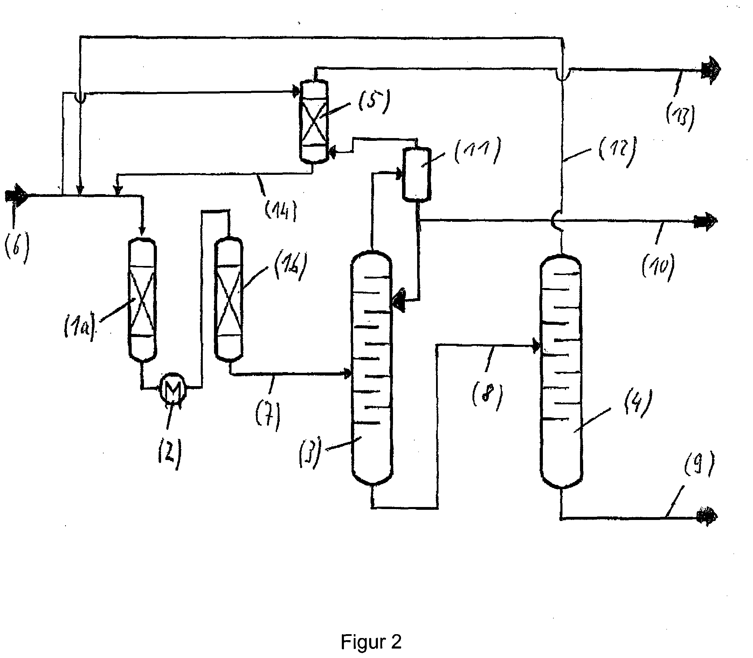

In Figur 2 wird eine Variante des erfindungsgemäßen Verfahrens schematisch dargestellt. Das Einsatz-Methanol (6) wird verdampft und überhitzt und dann einem ersten DME-Reaktor (1a) mit einer Temperatur von etwa 250 °C zugeführt. Die Gleichgewichtsreaktion von Methanol zu DME und Wasser findet an einem sauren Katalysator statt, bei einem Druck von ca. 12 - 14 bar(a) und mit einem Methanolumsatz von ca. 83 %. Das Reaktionsprodukt der ersten Reaktionsstufe wird durch Wärmeintegration von etwa 370 °C auf 250 °C zurückgekühlt, z.B. in einem Wärmeaustauscher (2), der das Produkt abkühlt und gleichzeitig den Einsatz-Methanoldampf (6) überhitzt. Das abgekühlte Reaktionsprodukt wird mit 250 °C in den zweiten DME-Reaktor (1b) eingespeist, wo sich weiteres Methanol umsetzt, aufgrund der günstigeren Gleichgewichtslage bei niedrigerer Temperatur. Das Reaktionsprodukt (7) verlässt den zweiten DME-Reaktor (1b) mit einer Temperatur von ca. 260 °C. Durch Verwendung des zweiten DME-Reaktors erhöht sich der Methanolumsatz insgesamt auf nunmehr ca. 88 %. Die Menge an nicht umgesetztem Methanol reduziert sich gleichzeitig um 30 %.

Nach (in Figur 2 nicht dargestellter) Kühlung des DME-Produkts (7) der zweiten Reaktionsstufe durch Wärmeaustausch, wiederum mit Einsatzmethanol (6) und/oder durch Beheizung eines Aufkochers oder durch Aufheizen von umgepumpten Strömen In der Nähe des Sumpfes einer der Kolonnen, wird dieses in die DME-Kolonne (3) geleitet. In dieser DME-Kolonne (3) wird das DME bei einem Druck von ca. 10 -12 bar(a) als flüssiges Kopfprodukt abgetrennt, bei Verwendung von Kühlwasser im Kopfkondensator, und einem Rückflußbehälter (11) zugeführt. Diesem wird flüssiges DME-Produkt (10) entnommen und aus der Anlage ausgeschleust bzw. ein Teil davon wird in die DME-Kolonne zurückgeführt. Die Gasphase aus dem Rückflußbehälter (11) wird einem DME-Absorber (5) zugeführt. Außerdem wird dem DME-Absorber (5) ein Teil des Einsatz-Methanols (6) zugeführt. Im DME-Absorber (5) werden nichtkondensierbare Gase, bestehend aus einer geringen Menge an Spaltgas (H₂, CO, CO₂ und CH₄) und DME, mit Methanol gewaschen, um das DME zurückzugewinnen und das flüssige Produkt (14) aus dem DME-Absorber (5) wird dem ersten DME-Reaktor (1a) zugeführt. Das Sumpfprodukt (8) der DME-Kolonne (3) wird in der Methanol-Kolonne (4), die bei geringem Oberdruck betrieben wird, in Methanol (12) und Wasser (9) aufgetrennt. In der Methanol-Kolonne (4) wird das nicht umgesetzte Methanol (12) zurückgewonnen, um dieses dem Prozess wieder zu zuführen.

## Patentansprüche

1. Verfahren zur Herstellung von Dimethylether durch katalytische Dehydratisierung von Methanol und destillative Aufarbeitung des Dehydratisierungsproduktes, wobei die katalytische Dehydratisierung in mindestens zwei in Reihe geschalteten Reaktionsstufen erfolgt, von denen zumindest die erste Reaktionsstufe adiabat betrieben wird und wobei zumindest zwischen der ersten und der zweiten Reaktionsstufe eine Kühlung des Reaktionsproduktes erfolgt, **dadurch gekennzeichnet, dass** drei in Reihe geschaltete adiabatisch betriebene Reaktionsstufen vorgesehen sind, zwischen der ersten und zweiten Reaktionsstufe flüssiges Methanol als Kühlmittel dem Reaktionsgemisch zugesetzt wird und dass zwischen der zweiten und dritten Reaktionsstufe die Kühlung des Reaktionsgemisches durch einen Wärmetauscher erfolgt, der Dämpfe des für die Dehydratisierung eingesetzten Methanols als Kühlmedium führt.

2. Verfahren nach Anspruch 1, **dadurch gekennzeichnet, dass** die Reaktionstemperatur in den Reaktionsstufen jeweils im Bereich von 200 bis 400°C, vorzugsweise von 250 bis 370°C, liegt.

3. Verfahren nach einem der Ansprüche 1 oder 2, **dadurch gekennzeichnet, dass** die Temperatur des Reaktionsgemisches zwischen den Reaktionsstufen auf 200 bis 300°C abgekühlt wird.

4. Verfahren nach einem der Ansprüche 1 bis 3, **dadurch gekennzeichnet, dass** die Reaktionsstufen in mindestens zwei in Reihe geschalteten adiabatisch betriebenen Reaktoren durchgeführt werden und dass zwischen den mindestens zwei adiabatisch betriebenen Reaktoren eine Kühlung des Reaktionsproduktes erfolgt.

5. Verfahren nach einem der Ansprüche 1 bis 4, **dadurch gekennzeichnet, dass** die Reaktionsstufen in einem adiabatisch betriebenen Reaktor durchgeführt werden, der mindestens zwei in Reihe geschaltete Katalysatorschüttungen aufweist, zwischen denen eine Kühlvorrichtung geschaltet ist, zur Zwischenkühlung des Reaktions-gemisches aus dem stromaufwärts liegenden Katalysatorbett.

6. Verfahren nach Anspruch 1, **dadurch gekennzeichnet, dass** ein adiabatisch betriebener Reaktor vorgesehen ist, der drei hintereinander geschaltete Katalysatorbetten aufweist, zwischen dem ersten und zweiten Katalysatorbett eine Vorrichtung zum Einbringen von flüssigem Methanol in das Reaktionsgemisch und zwischen dem zweiten und dritten Katalysatorbett einen Wärmetauscher, vorzugsweise einen Plattenwärmetauscher zum Kühlen des Reaktionsgemisches vor Eintritt in das dritte Katalysatorbett.

7. Verfahren nach einem der Ansprüche 1 bis 6, **dadurch gekennzeichnet, dass** für die Dehydratisierung ein saurer und fester Katalysator verwendet wird, vorzugsweise Aluminiumoxid.

8. Verfahren nach einem der Ansprüche 1 bis 7, **dadurch gekennzeichnet, dass** das aus der letzten Reaktionsstufe stammende Reaktionsgemisch in einer DME-Kolonne aufgearbeitet wird, indem Dimethylether aus dem Reaktiongemisch destillativ abgetrennt wird und ein Sumpfprodukt anfällt, wobei das Sumpfprodukt aus der DME-Kolonne in eine Methanol-Kolonne überführt wird, und dort destillativ in einen Methanolstrom und ein Wasser enthaltendes Sumpfprodukt aufgetrennt wird und wobei der Methanolstrom gegebenenfalls in eine oder mehrere DME-Reaktionsstufen zurückgeführt wird.

9. Verwendung einer Vorrichtung in einem Verfahren zur Herstellung von Dimethylether durch katalytische Dehydratisierung von Methanol gemäß einem der Ansprüche 1 bis 8, wobei diese Vorrichtung die Elemente umfasst:
A) mindestens zwei in Reihe geschaltete DME-Reaktoren (1a, 1b), von denen mindestens der erste DME-Reaktor adiabatisch betrieben wird,
B) zwischen mindestens dem ersten und dem zweiten DME-Reaktor angeordnete Kühlvorrichtung (2) für das Reaktionsgemisch aus dem stromaufwärts zur Kühlvorrichtung (2) gelegenen Reaktor (1a),
C) mit dem letzten Reaktor (1b) verbundene DME-Kolonne (3) zur Abtrennung des Dimethylethers aus dem Reaktionsgemisch, und
D) mit dem Sumpf der DME-Kolonne (3) verbundene Methanol-Kolonne (4) zur Auftrennung des vom Dimethylether befreiten Reaktionsgemischs in Methanol und Wasser, **dadurch gekennzeichnet, dass** die Kühlvorrichtung (2) ein Wärmetauscher ist, der Dämpfe des für die Dehydratisierung eingesetzten Methanols als Kühlmedium führt.

10. Verwendung einer Vorrichtung in einem Verfahren zur Herstellung von Dimethylether durch katalytische Dehydratisierung von Methanol gemäß einem der Ansprüche 1 bis 8, wobei diese Vorrichtung die Elemente umfasst:
A') mindestens einen DME-Reaktor, in dem mindestens zwei in Reihe geschaltete Reaktionsstufen angeordnet sind, von denen zumindest die erste Reaktionsstufe adiabatisch betrieben wird,
B') zwischen mindestens der ersten und der zweiten Reaktionsstufe angeordnete Kühlvorrichtung (2) für das Reaktionsgemisch aus der stromaufwärts zur Kühlvorrichtung gelegenen Reaktionsstufe,
C') mit der letzten Reaktionsstufe verbundene DME-Kolonne (3) zur Abtrennung des Dimethylethers aus dem Reaktionsgemisch, und
D) mit dem Sumpf der DME-Kolonne (3) verbundene Methanol-Kolonne (4) zur Auftrennung des vom Dimethylether befreiten Reaktionsgemischs in Methanol und Wasser, **dadurch gekennzeichnet, dass** die Kühlvorrichtung (2) ein Wärmetauscher ist, der Dämpfe des für die Dehydratisierung eingesetzten Methanols als Kühlmedium führt.

11. Verwendung nach Anspruch 10, **dadurch gekennzeichnet, dass** diese einen adiabat betriebenen DME-Reaktor aufweist, wobei der DME-Reaktor vorzugsweise ein vertikaler Schachtreaktor ist, in dem zwei in Reihe geschaltete Katalysator-schüttungen vorgesehen sind, sowie eine Kühlvorrichtung, die zur Zwischenkühlung des Reaktionsgemisches aus dem stromaufwärts liegenden Katalysatorbett dient.

12. Verwendung nach einem der Ansprüche 9 oder 10, **dadurch gekennzeichnet, dass** die Kühlvorrichtung (2) ein Wärmetauscher und/oder eine Vorrichtung zum Einleiten von Kühlflüssigkeit in das Reaktionsgemisch ist.

13. Verwendung nach einem der Ansprüche 9 oder 10, **dadurch gekennzeichnet, dass** das Methanol aus der Methanol-Kolonne (4) in den ersten adiabatisch betriebenen DME-Reaktor (1a) oder in die erste adiabatisch betriebene Reaktionsstufe des DME-Reaktors zurückgeführt wird.

## Claims

1. Process for preparing dimethyl ether by catalytic dehydration of methanol and work-up of the dehydration product by distillation, wherein the catalytic dehydration is carried out in at least two reaction stages connected in series, of which at least the first reaction stage is operated adiabatically and cooling of the reaction product is carried out at least between the first reaction stage and the second reaction stage, **characterized in that** three adiabatically operated reaction stages connected in series are provided, liquid methanol is added to the reaction mixture as cooling medium between the first and second reaction stages and **in that**, between the second and third reaction stages, the cooling of the reaction mixtures is effected by means of a heat exchanger through which vapor of the methanol used for the dehydration is conveyed as cooling medium.

2. Process according to Claim 1, **characterized in that** the reaction temperature in the reaction stages is in each case in the range from 200 to 400°C, preferably from 250 to 370°C.

3. Process according to either of Claims 1 and 2, **characterized in that** the temperature of the reaction mixture is brought down to from 200 to 300°C between the reaction stages.

4. Process according to any of Claims 1 to 3, **characterized in that** the reaction stages are carried out in at least two adiabatically operated reactors connected in series and **in that** cooling of the reaction product is carried out between the at least two adiabatically operated reactors.

5. Process according to any of Claims 1 to 4, **characterized in that** the reaction stages are carried out in an adiabatically operated reactor which has at least two catalyst beds connected in series between which a cooling apparatus is located for intermediate cooling of the reaction mixture from the catalyst bed located upstream.

6. Process according to Claim 1, **characterized in that** an adiabatically operated reactor which has three catalyst beds connected in series, a device for introducing liquid methanol into the reaction mixture between the first and second catalyst beds and a heat exchanger, preferably a plate heat exchanger, between the second and third catalyst beds in order to cool the reaction mixture before it enters the third catalyst bed is provided.

7. Process according to any of Claims 1 to 6, **characterized in that** an acidic and solid catalyst, preferably aluminum oxide, is used for the dehydration.

8. Process according to any of Claims 1 to 7, **characterized in that** the reaction mixture coming from the last reaction stage is worked up in a DME column in which dimethyl ether is separated off from the reaction mixture by distillation to leave a bottom product which is transferred from the DME column into a methanol column where it is separated by distillation into a methanol stream and a water-containing bottom product and the methanol stream is optionally recirculated to one or more of the DME reaction stages.

9. Use of an apparatus in a process for preparing dimethyl ether by catalytic dehydration of methanol according to any of Claims 1 to 8, wherein said apparatus comprises the elements:
A) at least two DME reactors (1a, 1b) connected in series, of which at least the first DME reactor is operated adiabatically,
B) a cooling apparatus (2) arranged between at least the first DME reactor and the second DME reactor for cooling the reaction mixture from the reactor (1a) located upstream of the cooling apparatus (2),
C) a DME column (3) connected to the last reactor (1b) for separating the dimethyl ether from the reaction mixture, and
D) a methanol column (4) connected to the bottom of the DME column (3) for separating the reaction mixture which has been freed of the dimethyl ether into methanol and water, **characterized in that** the cooling apparatus (2) is a heat exchanger through which vapor of the methanol used for the dehydration is conveyed as cooling medium.

10. Use of an apparatus in a process for preparing dimethyl ether by catalytic dehydration of methanol according to any of Claims 1 to 8, wherein said apparatus comprises the elements:
A') at least one DME reactor in which at least two reaction stages connected in series, of which at least the first reaction stage is operated adiabatically, are arranged,
B') a cooling apparatus (2) arranged between at least the first reaction stage and the second reaction stage for cooling the reaction mixture from the reaction stage located upstream of the cooling apparatus,
C') a DME column (3) connected to the last reaction stage for separating the dimethyl ether from the reaction mixture, and
D') a methanol column (4) connected to the bottom of the DME column (3) for separating the reaction mixture which has been freed of the dimethyl ether into methanol and water, **characterized in that** the cooling apparatus (2) is a heat exchanger through which vapor of the methanol used for the dehydration is conveyed as cooling medium.

11. Use according to Claim 10, **characterized in that** it comprises an adiabatically operated DME reactor, wherein the DME reactor is preferably a vertical shaft reactor, in which two catalyst beds connected in series are provided and a cooling apparatus which serves for intermediate cooling of the reaction mixture from the catalyst bed located upstream.

12. Use according to either Claim 9 or 10, **characterized in that** the cooling apparatus (2) is a heat exchanger and/or an apparatus for introducing cooling liquid into the reaction mixture.

13. Use according to either Claim 9 or 10, **characterized in that** the methanol from the methanol column (4) is recirculated to the first adiabatically operated DME reactor (1a) or to the first adiabatically operated reaction stage of the DME reactor.

## Revendications

1. Procédé pour la préparation de diméthyléther par déshydratation catalytique de méthanol et traitement par distillation du produit de déshydratation, la déshydratation catalytique étant effectuée dans au moins deux étages de réaction commutés en série, dont au moins le premier étage de réaction est exploité de manière adiabatique et un refroidissement du produit de réaction étant effectué au moins entre le premier et le deuxième étage de réaction, **caractérisé en ce que** trois étages de réaction commutés en série, exploités de manière adiabatique sont utilisés, du méthanol liquide est ajouté comme agent de refroidissement au mélange réactionnel entre le premier et le deuxième étage de réaction et **en ce que** le refroidissement du mélange réactionnel entre le deuxième et le troisième étage de réaction est effectué par un échangeur thermique qui guide des vapeurs du méthanol utilisé pour la déshydratation comme agent de refroidissement.

2. Procédé selon la revendication 1, **caractérisé en ce que** la température de réaction dans les étages de réaction se situe à chaque fois dans la plage de 200 à 400°C, de préférence de 250 à 370°C.

3. Procédé selon l'une quelconque des revendications 1 ou 2, **caractérisé en ce que** la température du mélange réactionnel entre les étages de réaction est refroidie à 200 jusqu'à 300°C.

4. Procédé selon l'une quelconque des revendications 1 à 3, **caractérisé en ce que** les étages de réaction sont effectués dans au moins deux réacteurs commutés en série, exploités de manière adiabatique et **en ce qu'**un refroidissement du produit de réaction est effectué entre lesdits au moins deux réacteurs exploités de manière adiabatique.

5. Procédé selon l'une quelconque des revendications 1 à 4, **caractérisé en ce que** les étages de réaction sont effectués dans un réacteur exploité de manière adiabatique, qui présente au moins deux lits catalytiques commutés en série entre lesquels est disposé un dispositif de refroidissement pour le refroidissement intermédiaire du mélange réactionnel sortant du lit catalytique situé en amont.

6. Procédé selon la revendication 1, **caractérisé en ce qu'**un réacteur exploité de manière adiabatique est utilisé, qui présente trois lits catalytiques disposés les uns derrière les autres, un dispositif pour introduire du méthanol liquide dans le mélange réactionnel entre le premier et le deuxième lit catalytique et un échangeur thermique, de préférence un échangeur thermique à plaques, entre le deuxième et le troisième lit catalytique, pour le refroidissement du mélange réactionnel avant l'entrée dans le troisième lit catalytique.

7. Procédé selon l'une quelconque des revendications 1 à 6, **caractérisé en ce qu'**un catalyseur acide et solide, de préférence de l'oxyde d'aluminium, est utilisé pour la déshydratation.

8. Procédé selon l'une quelconque des revendications 1 à 7, **caractérisé en ce que** le mélange réactionnel provenant du dernier étage de réaction est traité dans une colonne de DME, **en ce que** le diméthyléther est séparé par distillation du mélange réactionnel et un produit de fond est obtenu, le produit de fond étant transféré de la colonne de DME dans une colonne de méthanol et séparé dans celle-ci par distillation en un flux de méthanol et en un produit de fond contenant de l'eau et le flux de méthanol étant le cas échéant recyclé dans l'un ou plusieurs étages de réaction de DME.

9. Utilisation d'un dispositif dans un procédé pour la préparation de diméthyléther par déshydratation catalytique de méthanol selon l'une quelconque des revendications 1 à 8, ce dispositif comprenant les éléments :
A) au moins deux réacteurs de DME (1a, 1b) commutés en série, dont au moins le premier réacteur de DME est exploité de manière adiabatique,
B) un dispositif de refroidissement (2) disposé entre au moins le premier et le deuxième réacteur de DME pour le mélange réactionnel provenant du réacteur (la) situé en amont par rapport au dispositif de refroidissement (2),
C) une colonne de DME (3) reliée au dernier réacteur (1b) pour la séparation du diméthyléther à partir du mélange réactionnel et
D) une colonne de méthanol (4) reliée au fond de la colonne de DME (3) pour la séparation du mélange réactionnel libéré du diméthyléther en méthanol et en eau, **caractérisé**
**en ce que** le dispositif de refroidissement (2) est un échangeur thermique qui guide des vapeurs du méthanol utilisé pour la déshydratation comme agent de refroidissement.

10. Utilisation d'un dispositif dans un procédé pour la préparation de diméthyléther par déshydratation catalytique de méthanol selon l'une quelconque des revendications 1 à 8, ce dispositif comprenant les éléments :
A') au moins un réacteur de DME, dans lequel sont disposés au moins deux étages de réaction commutés en série, dont au moins le premier étage de réaction est exploité de manière adiabatique,
B') un dispositif de refroidissement (2) disposé entre au moins le premier et le deuxième étage de réaction pour le mélange réactionnel provenant de l'étage de réaction situé en amont par rapport au dispositif de refroidissement,
C') une colonne de DME (3) reliée au dernier étage de réaction pour la séparation du diméthyléther à partir du mélange réactionnel et
D) une colonne de méthanol (4) reliée au fond de la colonne de DME (3) pour la séparation du mélange réactionnel libéré du diméthyléther en méthanol et en eau, **caractérisé**
**en ce que** le dispositif de refroidissement (2) est un échangeur thermique qui guide des vapeurs du méthanol utilisé pour la déshydratation comme agent de refroidissement.

11. Utilisation selon la revendication 10, **caractérisée en ce que** celle-ci présente un réacteur de DME exploité de manière adiabatique, le réacteur de DME étant de préférence un réacteur à cuve vertical dans lequel sont disposés deux lits catalytiques commutés en série, ainsi qu'un dispositif de refroidissement qui sert au refroidissement intermédiaire du mélange réactionnel provenant du lit catalytique situé en amont.

12. Utilisation selon l'une quelconque des revendications 9 ou 10, **caractérisée en ce que** le dispositif de refroidissement (2) est un échangeur thermique et/ou un dispositif pour introduire du liquide de refroidissement dans le mélange réactionnel.

13. Utilisation selon l'une quelconque des revendications 9 ou 10, **caractérisée en ce que** le méthanol provenant de la colonne de méthanol (4) est recyclé dans le premier réacteur de DME (1a) exploité de manière adiabatique ou dans le premier étage de réaction exploité de manière adiabatique du réacteur de DME.
